# EUROPÄISCHE PATENTSCHRIFT

(11) **EP 1 335 728 B1**
(45) Veröffentlichungstag und Bekanntmachung des Hinweises auf die Patenterteilung: **23.03.2005**
(21) Anmeldenummer: 01989446.8
(22) Anmeldetag: 09.11.2001
(51) Int. Cl.: A61K 31/46, A61K 31/137, A61P 11/00, A61P 11/06

(54) **ARZNEIMITTELKOMPOSITIONEN AUF DER BASIS VON TIOTROPIUMSALZEN UND SALZEN DES SALMETEROLS**
MEDICAMENT COMPOSITIONS BASED ON TIOTROPIUM SALTS AND ON SALMETEROL SALTS
COMPOSITIONS DE MEDICAMENTS A BASE DE SELS DE TIOTROPIUM ET DE SELS DE SALMETEROL

(30) Priorität: 13.11.2000 DE 10056104
(43) Veröffentlichungstag der Anmeldung: 20.08.2003
(60) Teilanmeldung: 04028508.2
(73) Patentinhaber: Boehringer Ingelheim Pharma GmbH & Co.KG, 55216 Ingelheim am Rhein (DE)
(72) Erfinder: SCHMELZER, Christel, 55218 Ingelheim am Rhein (DE); NAGEL, Juergen, 55543 Bad Kreuznach (DE)
(86) Internationale Anmeldenummer: PCT/EP2001/012962
(87) Internationale Veröffentlichungsnummer: WO 2002/038154

(56) Entgegenhaltungen:
- WO-A-00/69468
- DE-A- 19 847 970
- BARNES P J: "Chronic obstructive pulmonary disease: new opportunities for drug development" TRENDS IN PHARMACOLOGICAL SCIENCES, ELSEVIER TRENDS JOURNAL, CAMBRIDGE, GB, Bd. 19, Nr. 10, Oktober 1998 (1998-10), Seiten 415-423, XP004156947 ISSN: 0165-6147
- REES P J: "BRONCHODILATORS IN THE THERAPY OF CHRONIC OBSTRUCTIVE PULMONARY DISEASE" EUROPEAN RESPIRATORY MONOGRAPH, EUROPEAN RESPIRATORY SOCIETY JOURNALS LTD., SHEFFIELD,, GB, Bd. 7, Nr. 7, 1998, Seiten 135-149, XP000937584 ISSN: 1025-448X

## Beschreibung

Die vorliegende Erfindung betrifft neurtige Arzneimittelkompositionen auf der Basis von Tiotropiumsalzen und Salzen des Salmeterols, Verfahren zu deren Herstellung sowie deren Verwendung bei der Therapie von Atemwegserkrankungen.

### Hintergrund der Erfindung

Die Verbindung Tiotropiumbromid, ein Salz des Tiotropiums, ist aus der Europäischen Patentanmeldung EP 418 716 A1 bekannt und weist die folgende chemische Struktur auf:

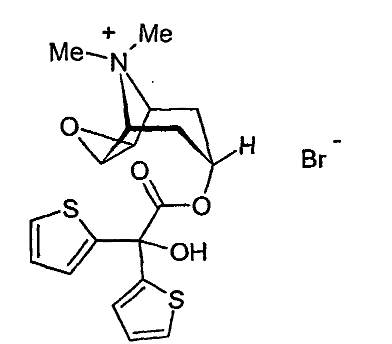

Diese Verbindung kann auch auch durch den chemischen Namen (1α,2β,4β,5α,7β)-7-[(hydroxydi-2-thienylacetyl)oxy]-9,9-dimethyl-3-oxa-9-azoniatricyclo[3.3.1.0^{2,4}] nonane-bromid bezeichnet werden und besitzt wertvolle pharmakologische Eigenschaften. Die Bezeichnung Tiotropium ist im Rahmen der vorliegenden Erfindung als Bezugnahme auf das freie Kation zu verstehen.

Sie, wie auch andere Salze des Tiotropiums, stellt ein hochwirksames Anticholinergikum dar und kann deshalb bei der Therapie von Asthma oder COPD (chronic obstructive pulmonary disease = chronisch obstruktive Lungenerkrankung) einen therapeutischen Nutzen entfalten.

Die Applikation von Tiotropiumsalzen erfolgt vorzugsweise auf inhalativem Wege. Hierbei können geeignete Inhaltionspulver, die in geeignete Kapseln (Inhaletten) abgefüllt mittels entsprechender Pulverinhalatoren appliziert werden, zum Einsatz kommen. Alternativ dazu kann eine inhalative Anwendung auch durch Applikation geeigneter Inhalationsaerosole erfolgen. Hierzu zählen auch pulverförmige Inhalationsaerosole, die beispielsweise HFA134a, HFA227 oder deren Gemisch als Treibgas enthalten. Die inhalative Applikation kann ferner mittels geeigneter Lösungen des Tiotropiumsalzes erfolgen.

### Detaillierte Beschreibung der Erfindung

Überraschenderweise wurde gefunden, daß ein unerwartet vorteilhafter therapeutischer Effekt, insbesondere ein synergistischer Effekt bei der Behandlung von entzündlichen oder obstruktiven Atemwegserkrankungen beobachtet wird, wenn ein oder mehrere Tiotropiumsalze (**1**) in Kombination mit ein oder mehreren Salmeterolsalzen (**2**) zur Anwendung gelangen.

Hierdurch lassen sich beispielsweise unerwünschte Nebenwirkungen, die häufig bei der Applikation von β-Mimetika, wie Salmeterol, am Menschen beobachtet werden deutlich verringern. Als zentrale Nebenwirkungen von β-Mimetika seien beispielsweise allgemeine Unruhe, Erregung, Schlaflosigkeit, Angst, Fingerzittern, Schweißausbrüche und Kopfschmerzen genannt.

Die Bezeichnung Tiotropium ist im Rahmen der vorliegenden Erfindung als Bezugnahme auf das freie Kation (**1'**) zu verstehen. Eine Bezugnahme auf Salmeterol ist im Rahmen der vorliegenden Erfindung als Bezugnahme auf die freie Base (**2'**) zu verstehen.

Die erfindungsgemäßen Wirkstoffkombinationen sind überraschenderweise ferner sowohl durch einen raschen Wirkungseintritt, als auch durch eine langandauemde Wirkdauer gekennzeichnet. Dies ist von hoher Bedeutung für das Wohlbefinden des Patienten, da er einerseits nach Applikation der Kombination eine rasche Verbesserung seines Zustands verspürt und andererseits aufgrund der langen Wirkdauer eine einmal pro Tag erfolgende Applikation ausreichend ist. Die vorstehend genannten Effekte werden sowohl bei gleichzeitiger Applikation innerhalb einer einzigen Wirkstoffformulierung als auch bei sukzessiver Applikation der beiden Wirkstoff in getrennten Formulierungen beobachtet. Erfindungsgemäß bevorzugt ist die gleichzeitige Applikation der beiden Wirkstoffbestandteile in einer einzigen Formulierung.

Ein Aspekt der vorliegenden Erfindung betrifft eine treibgasfreie Inhalationslösung, die als Lösemittel Wasser, Ethanol oder ein Gemisch aus Wasser und Ethanol enthält, gekennzeichnet durch einen Gehalt ein oder mehrere Tiotropiumsalze (**1**) und ein oder mehrere Salmeterolsalze (**2**), gegebenfalls in Form ihrer Solvate oder Hydrate, wobei das oder die Salze (**2**) des Salmeterols ausgewählt sind aus der Gruppe bestehend aus Hydrochlorid, Hydrobromid, Sulfat, Phosphat und Methansulfonat. Hierbei können die Wirkstoffe entweder gemeinsam in einer einzigen Darreichungsform oder in zwei getrennten Darreichungsformen enthalten sein. Erfindungsgemäß bevorzugt sind treibgasfreie Inhalationslösungen, die die Wirkstoffe **1** und **2** in einer einzigen Darreichungsform enthalten.

Ein weiterer Aspekt der vorliegenden Erfindung betrifft eine treibgasfreie Inhalationslösung, welche neben therapeutisch wirksamen Mengen von **1** und **2** einen pharmazeutisch verträglichen Hilfsstoff enthält. Ein Aspekt der vorliegenden Erfindung betrifft eine treibgasfreie Inhalationslösung, welche neben therapeutisch wirksamen Mengen von **1** und **2** keinen pharmazeutisch verträglichen Hilfsstoff enthält.

Die vorliegende Erfindung betrifft femer die Verwendung von **1** und **2** zur Herstellung eines therapeutisch wirksame Mengen von **1** und **2** enthaltenden Arzneimittels zur Behandlung von entzündlichen oder obstruktiven Atemwegserkrankungen, insbesondere von Asthma oder COPD durch simultane oder sukzessive Applikation.

Die vorliegende Erfindung zielt ferner auf die simultane oder sukzessive Verwendung therapeutisch wirksamer Dosen der Kombination vorstehender Arzneimittel **1** und **2** zur Behandlung von entzündlichen oder obstruktiven Atemwegserkrankungen, insbesondere von Asthma oder COPD.

Unter den im Rahmen der vorliegenden Erfindung einsetzbaren Tiotropiumsalzen **1** sind die Verbindungen zu verstehen, die neben Tiotropium als Gegenion (Anion) Chlorid, Bromid, lodid, Methansulfonat, para-Toluolsutfonat oder Methylsulfat enthalten. Im Rahmen der vorliegenden Erfindung sind von allen Tiotropiumsalzen das Methansulfonat, Chlorid, Bromid oder lodid bevorzugt, wobei dem Methansulfonat oder dem Bromid besondere Bedeutung zukommt. Von erfindungsgemäß herausragender Bedeutung ist das Tiotropiumbromid.

Unter Salzen des Salmeterols **2** werden erfindungsgemäß besonders bevorzugt pharmazeutisch verträgliche Salze verstanden, die ausgewählt sind aus Hydrochlorid und Sulfat, von denen die Sulfate besonders bevorzugt sind. Erfindungsgemäß von herausragender Bedeutung ist Salmeterol x ½ H₂SO₄.

In den erfindungsgemäßen Wirkstoffkombinationen aus **1** und **2** können die Bestandteile **1** und **2** in Form ihrer Enantiomere, Gemische der Enantiomere oder in Form der Racemate enthalten sein.

Die Verhältnisse, in denen die beiden Wirkstoffe **1** und **2** in die erfindungsgemäßen Wirkstoffkombinationen eingesetzt werden können, sind variabel. Die Wirkstoffe **1** und **2** können gegebenfalls in Form ihrer Solvate oder Hydrate vorliegen. Je nach Wahl der Salze **1** bzw. **2** variieren die im Rahmen der vorliegenden Erfindung einsetzbaren Gewichtsverhältnisse aufgrund des unterschiedlichen Molekulargewichts der verschiedenen Salzformen. Den nachfolgend angegebenen Gewichtsverhältnissen wurden daher das Tiotropiumkation **1'** sowie die freie Base des Salmeterols **2'** zugrunde gelegt. Die erfindungsgemäßen Wirkstoffkombinationen können **1'** und **2'** in Gewichtsverhältnissen enthalten, die in einem Bereich von 1:300 bis 30:1, bevorzugt von 1:230 bis 20:1, besonders bevorzugt von 1:150 bis 10:1, ferner bevorzugt von 1:50 bis 5:1, besonders bevorzugt von 1:35 bis 2:1. Erfindungsgemäß von besonderem Interesse sind Arzneimittel, enthaltend die Kombination aus **1'** und **2'** in einem Gewichtsverhältnis im Bereich von 1:25 bis 1:1. bevorzugt in einem Bereich von 1:10 bis 1:2, besonders bevorzugt in einem Bereich von 1:5 bis 1:2,5.

Beispielsweise und ohne den Umfang der Erfindung darauf zu beschränken, können bevorzugte erfindungsgemäße Kombinationen aus **1** und **2** Tiotropium **1'** und Salmeterol **2'** in den folgenden Gewichtsverhältnissen enthalten: 1:40; 1:20; 1:11,1; 1:10; 1:5,6; 1:5; 1:2,8; 1:2,5; 1:1,4; 1:1,25; 1,44:1, 1,6:1.

Die Anwendung der erfindungsgemäßen Arzneimittel enthaltend die Kombinationen aus **1** und **2** erfolgt üblicherweise so, daß Tiotropium **1'** und Salmeterol **2'** gemeinsam in Dosierungen von 0,01 bis 10000µg, bevorzugt von 0,1 bis 2000µg, besonders bevorzugt von 1 bis 1000µg, femer bevorzugt von 5 bis 500µg, erfindungsgemäß bevorzugt von 10 bis 200µg, bevorzugt von 20 bis 100µg, höchst bevorzugt von 30 bis 70µg pro Einmalgabe.
Beispielsweise enthalten erfindungsgemäße Kombinationen aus **1** und **2** eine solche Menge an Tiotropium **1'** und Salmeterol **2'**, daß die Gesamtdosierung pro Einmalgabe 30µg, 35µg, 45µg, 55µg, 60µg, 65µg, 90µg, 105µg, 110µg, 110µg, 140µg oder ähnliches beträgt. Bei diesen Dosierungsbereichen sind die Wirkstoffe **1'** und **2'** in den vorhergehend beschriebenen Gewichtsverhältnissen enthalten.

Beispielsweise und ohne den Umfang der Erfindung darauf zu beschränken, können die erfindungsgemäßen Kombinationen aus **1** und **2** eine solche Menge an Tiotropium **1'** und Salmeterol **2'** enthalten, daß pro Einmalgabe 5µg **1'** und 25µg **2'**, 5µg **1'** und 50µg **2'**, 5µg **1'** und 100µg **2'**, 5µg **1'** und 200µg **2'**, 10µg **1'** und 25µg **2'**, 10µg **1'** und 50µg **2'**, 10µg **1'** und 100µg **2'**, 10µg **1'** und 200µg **2'**, 18µg **1'** und 25µg **2'**, 18µg **1'** und 50µg **2'**, 18µg **1'** und 100µg **2',** 18µg **1'** und 200µg **2'**, 20µg **1'** und 25µg **2'**, 20µg **1'** und 50µg **2'**, 20µg **1'** und 100µg **2'**, 20µg **1'** und 200µg **2'**, 36µg **1'** und 25µg **2'**, 36µg **1'** und 50µg **2'**, 36µg **1'** und 100µg **2'**, 36µg **1'** und 200µg **2'**, 40µg **1'** und 25µg **2'**, 40µg **1'** und 50µg **2'**, 40µg **1'** und 100µg **2'** oder 40µg **1'** und 200µg **2'** appliziert werden.

Wird als erfindungsgemäß bevorzugte Kombination aus **1** und **2** die Wirkstoffkombination verwendet, in der **1** Tiotropiumbromid bedeutet und in der **2** für Salmeterol x ½H₂SO₄ steht, entsprechen die vorstehend beispielhaft genannten pro Einmalgabe applizierten Wirkstoffmengen von **1'** und **2'** den nachfolgenden pro Einmalgabe applizierten Mengen an **1** und **2**: 6µg **1** und 27,9µg **2**, 6µg **1** und 55,9µg **2,** 6µg **1** und 111,8µg **2,** 6µg **1** und 223,6µg **2**, 12µg **1** und 27,9µg **2**, 12µg **1** und 55,9µg **2**, 12µg **1** und 111,8µg **2**, 12µg **1** und 223,6µg **2**, 21,7µg **1** und 27,9µg **2**, 21,7µg **1** und 55,9µg **2**, 21,7µg **1** und 111,8µg **2**, 21,7µg **1** und 223,6µg **2**, 24,1µg **1** und 27,9µg **2**, 24,1µg **1** und 55,9µg **2**, 24,1µg **1** und 111,8µg **2**, 24,1µg **1** und 223,6µg **2,** 43,3µg **1** und 27,9µg **2,** 43,3µg **1** und 55,9µg **2**, 43,3µg **1** und 111,8µg **2**, 43,3µg **1** und 223,6µg **2**, 48,1µg **1** und 27,9µg **2**, 48,1µg **1** und 55,9µg **2,** 48,1µg **1** und 111,8µg **2** oder 48,1µg **1** und 223,6µg **2**.

Wird in der erfindungsgemäß bevorzugten Kombination aus **1** und **2**, in der **2** für Salmeterol x ½H₂SO₄ steht als **1** beispielsweise das Tiotropiumbromidmonohydrat eingesetzt, entsprechen die vorstehend beispielhaft genannten pro Einmalgabe applizierten Wirkstoffmengen von **1'** und **2'** den nachfolgenden pro Einmalgabe applizierten Mengen an **1** und **2:** 6,2µg **1** und 27,9µg **2,** 6,2µg **1** und 55,9µg **2,** 6,2µg **1** und 111,8µg **2**, 6,2µg **1** und 223,6µg **2**, 12,5µg **1** und 27,9µg **2**, 12,5µg **1** und 55,9µg **2**, 12,5µg **1** und 111,8µg **2**, 12,5µg **1** und 223,6µg **2**, 22,5µg **1** und 27,9µg **2**, 22,5µg **1** und 55,9µg **2,** 22,5µg **1** und 111,8µg **2**, 22,5µg **1** und 223,6µg **2**, 25µg **1** und 27,9µg **2**, 25µg **1** und 55,9µg **2,** 25µg **1** und 111,8µg **2**, 25µg **1** und 223,6µg **2**, 45µg **1** und 27,9µg **2**, 45µg **1** und 55,9µg **2,** 45µg **1** und 111,8µg **2**, 45µg **1** und 223,6µg **2**, 50µg **1** und 27,9µg **2**, 50µg **1** und 55,9µg **2**, 50µg **1** und 111,8µg **2** oder 50µg **1** und 223,6µg **2**.

Die Applikation der erfindungsgemäßen Wirkstoffkombinationen aus **1** und **2** erfolgt bevorzugt auf inhalativem Wege. Hierzu müssen die Bestandteile **1** und **2** in inhalierbaren Darreichungsformen bereitgestellt werden.
Als inhalierbare Darreichungsformen kommen erfindungsgemäß treibgasfreie Inhalationslösungen in Betracht. Im Rahmen der vorliegenden Erfindung sind von dem Begriff treibgasfreie Inhalationslösungen auch Konzentrate oder sterile, gebrauchsfertige Inhalationslösungen umfaßt. Die erfindungsgemäßen Darreichungsformen können die Wirkstoffkombination aus **1** und **2** entweder gemeinsam in einer oder in zwei getrennten Darreichungsformen enthalten. Diese im Rahmen der vorliegenden Erfindung einsetzbaren Darreichungsformen werden im nachfolgenden Teil der Beschreibung detailliert beschrieben.

### Treibgasfreie Inhaltionslösungen enthaltend die erfindungsgemäßen Wirkstoffkombinationen aus 1 und 2:

Besonders bevorzugt erfolgt die Applikation der erfindungsgemäßen Wirkstoffkombination in Form von treibgasfreien Inhalationslösungen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Der relative Anteil an Ethanol gegenüber Wasser ist nicht begrenzt, bevorzugt liegt die maximale Grenze jedoch bei bis 70 Volumenprozent, insbesondere bei bis zu 60 Volumenprozent und besonders bevorzugt bei bis zu 30 Volumenprozent. Die restlichen Volumenprozente werden von Wasser aufgefüllt. Bevorzugtes Lösungsmittel ist Wasser ohne ethanolischen Zusatz. Die **1** und **2** , getrennt oder gemeinsam enthaltenden Lösungen werden mit geeigneten Säuren auf einen pH-Wert von 2 bis 7, bevorzugt von 2 bis 5, besonders bevorzugt von 2,5 bis 3,5 eingestellt. Höchst bevorzugt weisen erfindungsgemäße Inhaltionslösungen, die gemeinsam **1** und **2** enthalten einen pH-Wert von etwa 2,9 auf. Zur Einstellung dieses pH-Werts können Säuren ausgewählt aus anorganischen oder organischen Säuren Verendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit dem Wirkstoff oder im Fall von Kombinationspräparaten mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

Erfindungsgemäß kann in der vorliegenden Formulierung auf den Zusatz von Editinsäure (EDTA) oder einem der bekannten Salze davon, Natriumedetat, als Stabilisator oder Komplexbildner verzichtet werden.
Andere Ausführungsformen beinhalten diese Verbindung(en).
In einer solchen bevorzugten Ausführungsform liegt der Gehalt bezogen auf Natriumedetat unter 100 mg /100 ml, bevorzugt unter 50 mg/100 ml, besonders beovorzugt unter 20 mg/100 ml.
Generell sind solche Inhaltionslösungen bevorzugt, in denen der Gehalt an Natriumedetat bei 0 bis 10mg/100ml liegt.

Den erfindungsgemäßen treibgasfreien Inhaltionslösungen können Co-Solventien und/oder weitere Hilfsstoffe zugesetzt werden.
Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester.
Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu denZusatzstoffen zählen auch pharmakologisch unbedenkliche Salze wie beispielsweise Natriumchlorid als Isotonantien.

Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.
Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/100ml, besondersbevorzugt zwischen 5 und 20 mg/100ml enthalten.

Bevorzugte Formulierungen enthalten außer dem Lösungsmittel Wasser und der Wirkstoffkombination aus **1** und **2** nur noch Benzalkoniumchlorid und Natriumedetat. In einer anderen bevorzugten Ausführungsform wird auf Natriumedetat verzichtet.

Zur Applikation der erfindungsgemäßen treibgasfreien Inhaltionslösungen sind besonders solche Inhalatoren, die eine kleine Menge einer flüssigen Formulierung in der therapeutisch notwendigen Dosierung binnen weniger Sekunden in ein therapeutisch-inhalativ geeignetes Aerosol vernebeln können. Im Rahmen der vorliegenden Erfindung sind solche Vemebler bevorzugt, bei denen bereits eine Menge von weniger als 100 µL, bevorzugt weniger als 50 µL, besonders bevorzugt zwischen 20 und 30 µL Wirkstofflösung mit bevorzugt einem Hub zu einem Aerosol mit einer durchschnittlichen Teilchengröße von weniger als 20 µm, bevorzugt weniger als 10 µm, so vemebelt werden können, daß der inhalierbare Anteil des Aerosols bereits der therapeutisch wirksamen Menge entspricht.

Eine derartige Vorrichtung zur treibgasfreien Verabreichung einer dosierten Menge eines flüssigen Arzneimittels zur inhalativen Anwendung, wird beispielsweise in der internationalen Patentanmeldung WO 91/14468 als auch in der WO 97/12687 (insbesondere Figuren 6a und 6b) ausführlich beschrieben. Die dort beschriebenen Vernebler (Devices) sind auch unter der Bezeichnung Respimat® bekannt.

Dieser Vernebler (Respimat®) kann vorteilhaft zur Erzeugung der erfindungsgemäßen inhalierbaren Aerosole enthaltend die Wirkstoffkombination aus **1** und **2** eingesetzt werden. Aufgrund seiner zylinderähnlichen Form und einer handlichen Größe von weniger als 9 bis 15 cm in der Länge und 2 bis 4 cm in der Breite kann dieses Device jederzeit vom Patienten mitgeführt werden. Der Vemebler versprüht ein definiertes Volumen der Arzneimittelformulierung unter Anwendung hoher Drücke durch kleine Düsen, so daß inhalierbare Aerosole.entstehen.

Im wesentlichen besteht der bevorzugte Zerstäuber aus einem Gehäuseoberteil, einem Pumpengehäuse, einer Düse, einem Sperrspannwerk, einem Federgehäuse, einer Feder und einem Vorratsbehälter, gekennzeichnet durch
- ein Pumpengehäuse, das im Gehäuseoberteil befestigt ist; und das an seinem einen Ende einen Düsenkörper mit der Düse bzw. Düsenanordnung trägt,
- einen Hohlkolben mit Ventilkörper,
- einen Abtriebsflansch, in dem der Hohlkolben befestigt ist, und der sich im Gehäuseoberteil befindet,
- ein Sperrspannwerk, das sich im Gehäuseoberteil befindet,
- ein Federgehäuse mit der darin befindlichen Feder, das am Gehäuseoberteil mittels eines Drehlagers drehbar gelagert ist,
- ein Gehäuseunterteil, das auf das Federgehäuse in axialer Richtung aufgesteckt ist.

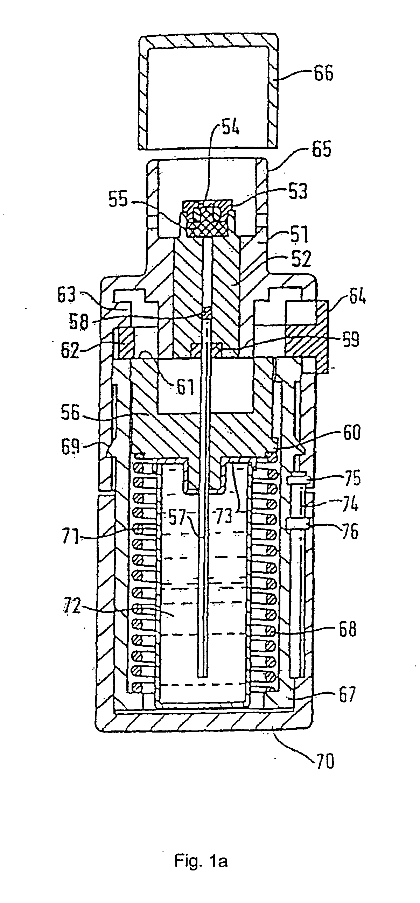

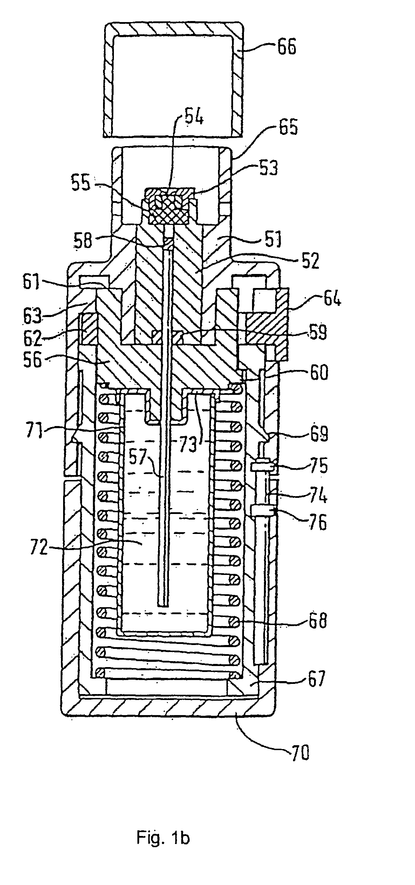

Der Hohlkolben mit Ventilkörper entspricht einer in der WO 97/12687 offenbarten Vorrichtung. Er ragt teilweise in den Zylinder des Pumpengehäuses hinein und ist im Zylinder axial verschiebbar angeordnet. Insbesondere wird auf die Figuren 1-4 - insbesondere Figur 3 - und die dazugehörigen Beschreibungsteile Bezug genommen. Der Hohlkolben mit Ventilkörper übt auf seiner Hochdruckseite zum Zeitpunkt des Auslösens der Feder einen Druck von 5 bis 60 Mpa (etwa 50 bis 600 bar), bevorzugt 10 bis 60 Mpa (etwa 100 bis 600 bar) auf das Fluid, die abgemessene Wirkstofflösung aus. Dabei werden Volumina von 10 bis 50 Mikroliter bevorzugt, besonders bevorzugt sind Volumina von 10 bis 20 Mikroliter, ganz besonders bevorzugt ist ein Volumen von 15 Mikroliter pro Hub.

Der Ventilkörper ist bevorzugt an dem Ende des Hohlkolbens angebracht, das dem Düsenkörper zugewandt ist.

Die Düse im Düsenkörper ist bevorzugt mikrostrukturiert, d.h. durch Mikrotechnik hergestellt. Mikrostrukturierte Düsenkörper sind beispielsweise in der WO-94/07607 offenbart; auf diese Schrift wird hiermit inhaltlich Bezug genommen, insbesondere auf Figur 1 und deren Beschreibung.
Der Düsenkörper besteht z.B. aus zwei fest miteinander verbundenen Platten aus Glas und/oder Silizium, von denen wenigstens eine Platte einen oder mehrere mikrostrukturierte Kanäle aufweist, die die Düseneinlaßseite mit der Düsenauslaßseite verbinden. Auf der Düsenauslaßseite ist mindestens eine runde oder nicht-runde Öffnung von 2 bis 10 Mikrometer Tiefe und 5 bis 15 Mikrometern Breite, wobei die Tiefe bevorzugt bei 4, 5 bis 6,5 Mikrometern und die Länge bei 7 bis 9 Mikrometern beträgt.
Im Fall von mehreren Düsenöffnungen, bevorzugt sind zwei, können die Strahlrichtungen der Düsen im Düsenkörper parallel zueinander verlaufen oder sie sind in Richtung Düsenöffnung gegeneinander geneigt. Bei einem Düsenkörper mit mindestens zwei Düsenöffnungen auf der Auslaßseite können die Strahlrichtungen mit einem Winkel von 20 Grad bis 160 Grad gegeneinander geneigt sein, bevorzugt wird ein Winkel von 60 bis 150 Grad, insbesondere bevorzugt 80 bis 100°.
Die Düsenöffnungen sind bevorzugt in einer Entfernung von 10 bis 200 Mikrometern angeordnet, stärker bevorzugt in einer Entfernung von 10 bis 100 Mikrometer, besonders bevorzugt 30 bis 70 Mikrometer. Am stärksten bevorzugt sind 50 Mikrometer.
Die Strahlrichtungen treffen sich dementsprechend in der Umgebung der Düsenöffnungen.

Die flüssige Arzneimittelzubereitung trifft mit einem Eingangsdruck von bis zu 600 bar, bevorzugt 200 bis 300 bar auf den Düsenkörper und wird über die Düsenöffnungen in ein inhalierbares Aerosol zerstäubt. Die bevorzugten Teilchen- bzw. Tröpfchengrößen des Aerosols liegen bei bis zu 20 Mikrometern, bevorzugt 3 bis 10 Mikrometern.

Das Sperrspannwerk enthält eine Feder, bevorzugt eine zylindrische schraubenförmige Druckfeder, als Speicher für die mechanische Energie. Die Feder wirkt auf den Abtriebsflansch als Sprungstück, dessen Bewegung durch die Position eines Sperrglieds bestimmt wird. Der Weg des Abtriebsflansches wird durch einen oberen und einen unteren Anschlag präzise begrenzt. Die Feder wird bevorzugt über ein kraftübersetzendes Getriebe, z.B. ein Schraubschubgetriebe, durch ein äußeres Drehmoment gespannt, das beim Drehen des Gehäuseoberteils gegen das Federgehäuse im Gehäuseunterteil erzeugt wird. In diesem Fall enthalten das Gehäuseoberteil und der Abtriebsflansch ein ein- oder mehrgängiges Keilgetriebe.

Das Sperrglied mit einrückenden Sperrflächen ist ringförmig um den Abtriebsflansch angeordnet. Es besteht z.B. aus einem in sich radial elastisch verformbaren Ring aus Kunststoff oder aus Metall. Der Ring ist in einer Ebene senkrecht zur Zerstäuberachse angeordnet. Nach dem Spannen der Feder schieben sich die Sperrflächen des Sperrgliedes in den Weg des Abtriebsflansches und verhindern das Entspannen der Feder. Das Sprerrglied wird mittels einer Taste ausgelöst. Die Auslösetaste ist mit dem Sperrglied verbunden oder gekoppelt. Zum Auslösen des Sperrspannwerkes wird die Auslösetaste parallel zur Ringebene, und zwar bevorzugt in den Zerstäuber hinein, verschoben; dabei wird der verformbare Ring in der Ringebene verformt. Konstruktive Details des.Sperrspannwerkes sind in der WO 97/20590 beschrieben.

Das Gehäuseunterteil wird in axialer Richtung über das Federgehäuse geschoben und verdeckt die Lagerung, den Antrieb der Spindel und den Vorratsbehälter für das Fluid.

Beim Betätigen des Zerstäubers wird das Gehäuseobereil gegen das Gehäuseunterteil gedreht, wobei das Gehäuseunterteil das Federgehäuse mitnimmt. Dabei wird die Feder über das Schraubschubgetriebe zusammengedrückt und gespannt, und das Sperrwerk rastet selbsttätig ein. Der Drehwinkel ist bevorzugt ein ganzzahliger Bruchteil von 360 Grad, z.B. 180 Grad. Gleichzeitig mit dem Spannen der Feder wird das Abtriebsteil im Gehäuseoberteil um einen vorgegebenen Weg verschoben, der Hohlkolben wird innerhalb des Zylinders im Pumpengehäuse zurückgezogen, wodurch eine Teilmenge des Fluids aus dem Vorratsbehälter in den Hochdruckraum vor der Düse eingesaugt wird.

In den Zerstäuber können gegebenenfalls nacheinander mehrere das zu zerstäubende Fluid enthaltende austauschbare Vorratsbehälter eingeschoben und benutzt werden. Der Vorratsbehälter enthält die erfindungsgemäße wässerige Aerosolzubereitung.

Der Zerstäubungsvorgang wird durch leichtes Eindrücken der Auslösetaste eingeleitet. Dabei gibt das Sperrwerk den Weg für das Abtriebsteil frei. Die gespannte Feder schiebt den Kolben in den Zylinder des Pumpengehäuses hinein. Das Fluid tritt aus der Düse des Zerstäubers in zerstäubter Form aus.

Weitere konstruktive Details sind in den PCT-Anmeldungen WO 97/12683 und WO 97/20590 offenbart, auf die hiermit inhaltlich Bezug genommen wird.

Die Bauteile des Zerstäubers (Vemeblers) sind aus einem der Funktion entsprechend geeignetem Material. Das Gehäuse des Zerstäubers und - so weit es die Funktion erlaubt - auch andere Teile sind bevorzugt aus Kunststoff, z.B. im Spritzgießverfahren, hergestellt. Für medizinische Zwecke werden physiologisch unbedenkliche Materialien verwendet.

In den Figuren 1a/b, die identisch sind mit den Figuren 6 a/b der WO 97/12687, ist der Vemebler (Respimat®) beschrieben, mit dem die erfindungsgemäßen wäßrigen Aerosolzubereitungen vorteilhaft inhaliert werden können.

Figur 1 a zeigt einen.Längsschnitt durch den Zerstäuber bei gespannter Feder, Figur 2 b zeigt einen Längsschnitt durch den Zerstäuber bei entspannter Feder.

Das Gehäuseoberteil (51) enthält das Pumpengehäuse (52), an dessen Ende der Halter (53) für die Zerstäuberdüse angebracht ist. In dem Halter befindet sich der Düsenkörper (54) und ein Filter (55). Der im Abtriebsflansch (56) des Sperrspannwerkes befestigte Hohlkolben (57) ragt teilweise in den Zylinder des Pumpengehäuses hinein. An seinem Ende trägt der Hohlkolben den Ventilkörper (58). Der Hohlkolben ist mittels der Dichtung (59) abgedichtet. Innerhalb des Gehäuseoberteils befindet sich der Anschlag (60), an dem der Abtriebsflansch bei entspannter Feder anliegt. Am Abtriebsflansch befindet sich der Anschlag (61), an dem der Abtriebsflansch bei gespannter Feder anliegt. Nach dem Spannen der Feder schiebt sich das Sperrglied (62) zwischen den Anschlag (61) und eine Abstützung (63) im Gehäuseoberteil. Die Auslösetaste (64) steht mit dem Sperrglied in Verbindung. Das Gehäuseobereil endet im Mundstück (65) und ist mit der aufsteckbaren Schutzkappe (66) verschlossen.

Das Federgehäuse (67) mit Druckfeder (68) ist mittels der Schnappnasen (69) und Drehlager am Gehäuseoberteil drehbar gelagert. Über das Federgehäuse ist das Gehäuseunterteil (70) geschoben. Innerhalb des Federgehäuses befindet sich der austauschbare Vorratsbehälter (71) für das zu zerstäubende Fluid (72). Der Vorratsbehälter ist mit dem Stopfen (73) verschlossen, durch den der Hohlkolben in den Vorratsbehälter hineinragt und mit seinem Ende in das Fluid (Vorrat an Wirkstofflösung) eintaucht.

In der Mantelfläche des Federgehäuses ist die Spindel (74) für das mechanische Zählwerk angebracht. An dem Ende der Spindel, das dem Gehäuseoberteil zugewandt ist, befindet das Antriebsritzel (75). Auf der Spindel sitzt der Reiter (76).

Der oben beschriebene Vernebler ist geeignet, die erfindungsgemäßen Aerosolzubereitungen zu einem für die Inhalation geeignetem Aerosol zu vernebeln.

Wird die erfindungsgemäße Formulierung mittels der vorstehend beschriebenen Technik (Respimat®) vemebelt, sollte die ausgebrachte Masse bei wenigstens 97%, bevorzugt wenigstens 98% aller Betätigungen des Inhalators (Hube) einer definierten Menge mit einem Toleranzbereichs von maximal 25%, bevorzugt 20% dieser Menge entsprechen. Bevorzugt werden pro Hub zwischen 5 und 30 mg Formulierung als definierte Masse ausgebracht, besonders bevorzugt zwischen 5 und 20 mg.

Die erfindungsgemäße Formulierung kann jedoch auch mittels anderer als der vorstehend beschriebenen Inahalatoren, beispielsweise Jet-Stream-Inhalatoren, vernebelt werden.

Dementsprechend betrifft ein weiterer Aspekt der vorliegenden Erfindung Arzneimittel in Form von wie vorstehend beschriebenen treibgasfreien Inhaltionslösungen in Verbindung mit einer zur Verabreichung dieser Lösungen geeigneten Vorrichtung, bevorzugt in Verbindung mit dem Respimat®. Bevorzugt zielt die vorliegende Erfindung auf treibgasfreie Inhaltionslösungen gekennzeichnet durch die erfindungsgemäßen Wirkstoffkombination aus **1** und **2** in Verbindung mit der unter der Bezeichnung Respimat® bekannten Vorrichtung. Femer betrifft die vorliegende Erfindung vorstehend genannte Vorrichtungen zur Inhalation, bevorzugt den Respimat®, dadurch gekennzeichnet, daß sie vorstehend beschriebene erfindungsgemäße treibgasfreie Inhaltionslösungen enthalten.

Die erfindungsgemäßen treibgasfreien Inhalationslösungen können neben den vorstehend, zur Applikation im Respimat vorgesehenen Lösungen auch als Konzentrate oder sterile gebrauchsfertige Inhalationslösungen vorliegen. Aus den Konzentraten lassen sich beispielsweise durch Zugabe von isotonischen Kochsalzlösungen gebrauchsfertige Inhalationslösungen generieren. Sterile gebrauchsfertige Inhalationslösungen können mittels energiebetriebener Stand- oder transportabler Vernebler, die inhalierbare Aerosole mittels Ultraschall oder Druckluft nach dem Venturiprinzip oder anderen Prinzipien erzeugen, appliziert werden.

Dementsprechend betrifft ein weiterer Aspekt der vorliegenden Erfindung Arzneimittel in Form von wie vorstehend beschriebenen treibgasfreien Inhaltionslösungen, die als Konzentrate oder sterile gebrauchsfertige Lösungen vorliegen, in Verbindung mit einer zur Verabreichung dieser Lösungen geeigneten Vorrichtung, dadruch gekennzeichnet, daß s sich bei dieser Vorrichtung um einen energiebetriebenen Stand- oder transportablen Vernebler handelt, der inhalierbare Aerosole mittels Ultraschall oder Druckluft nach dem Venturiprinzip oder anderen Prinzipien erzeugt.

Die folgenden Beispiele dienen einer weitergehenden Erläuterung der vorliegenden Erfindung, ohne den Umfang der Erfindung allerdings auf die nachfolgenden beispielhaften Ausführungsformen zu beschränken.

### Ausgangsmaterialien

### Tiotropiumbromid:

Das in den nachfolgenden Formulierungsbeispielen eingesetzte Tiotropiumbromid kann wie in der Europäischen Patentanmeldung EP 418 716 A1 beschrieben, erhalten werden.

Zur Herstellung der erfindungsgemäßen Inhalationslösungen kann ebenfalls kristallines Tiotropiumbromidmonohydrat eingesetzt werden. Dieses kristalline Tiotropiumbromidmonohydrat ist gemäß nachfolgend beschriebener Vorgehensweise erhältlich.
In einem geeigneten Reaktionsgefäß werden in 25,7 kg Wasser 15,0 kg Tiotropiumbromid eingetragen. Die Mischung wird auf 80-90°C erhitzt und bei gleichbleibender Temperatur solange gerührt, bis eine klare Lösung entsteht. Aktivkohle (0,8 kg), wasserfeucht, wird in 4,4 kg Wasser aufgeschlämmt, diese Mischung in die Tiotropiumbromid-haltige Lösung eingetragen und mit 4,3 kg Wasser nachgespült. Die so erhaltene Mischung wird wenigstens 15 min bei 80-90°C gerührt und anschließend über einen beheizten Filter in einen auf 70°C Manteltemperatur vorgewärmten Apparat filtriert. Der Filter wird mit 8,6 kg Wasser nachgespült. Der Apparateinhalt wird mit 3-5°C pro 20 Minuten auf eine Temperatur von 20-25°C abgekühlt. Mit Kaltwasserkühlung wird der Apparat auf 10-15°C weiter abgekühlt und die Kristallisation durch mindestens einstündiges Nachrühren vervollständigt. Das Kristallisat wird über einen Nutschentrockner isoliert, der isolierte Kristallbrei mit 9 L kaltem Wasser (10-15°C) und kaltem Aceton (10-15°C) gewaschen. Die erhaltenen Kristalle werden bei 25°C über 2 Stunden im Stickstoffstrom getrocknet. Ausbeute : 13,4 kg Tiotropiumbromidmonohydrat (86 % d. Th.)

Das so erhaltene kristalline Tiotropiumbromidmonohydrat wird nach bekannten Verfahren mikronisiert, um den Wirkstoff in Form der mittleren Teilchengröße bereitzustellen, die den erfindungsgemäßen Spezifikationen entspricht.

### Salmeterol x ½H₂SO₄:

Wird in den nachfolgenden Ausführungsbeispielen auf Salmeterol x % H₂SO₄ Bezug genommen, so wurde dieses wie folgt erhalten:
Eine Suspension aus 2.5 g (4.15 mmol) Salmeterolxinafoat wird in 6 ml Ethanol gelöst. Unter Rühren wird eine Lösung von 0.14 ml 98%iger Schwefelsäure langsam zu der Suspension gegeben. Es wird bis zur vollständigen Lösung auf 35-40 °C erwärmt. Anschließend wird mit 10 ml Diethylether verdünnt und die Lösung mit Salmeterotsulfat angeimpft. Das Salmeterolsutfat wird nach 1.5 Stunden abgesaugt und mit je 20 ml kaltem Ethanol, Aceton und Diethylether gewaschen.
Man erhält 1.5 g (78%) Salmeterol-½-sulfat

### Formulierungsbeispiele

### Treibgasfreie Inhaltionslösungen:

1) Lösung zur Anwendung im Respimat®:

| **Bestandteile** | **mg/100mL** |
|---|---|
| Tiotropiumbromid | 148,5 |
| Salmeterol x ½ H₂SO₄ | 276,7 |
| Benzalkoniumchlorid | 10 |
| Natriumedetat | 10 |
| Salzsäure (aq) | ad pH 2,9 |
| Wasser | ad 100mL |

Verwendung der Lösung im Respimat führt zu einer Dosierung von 10µg pro Dosierung **1** und 25µg/Dosierung **2**.
2) Lösung zur Anwendung im Respimat®:

| **Bestandteile** | **mg/100mL** |
|---|---|
| Tiotropiumbromid | 148,5 |
| Salmeterol x ½ H₂SO₄ | 276,7 |
| Benzalkoniumchlorid | 10 |
| Salzsäure (aq) | ad pH 2,9 |
| Wasser | ad 100mL |

Verwendung der Lösung im Respimat führt zu einer Dosierung von 10µg pro Dosierung **1** und 25µg/Dosierung **2.**
3) Lösung zur Anwendung im Respimat®:

| **Bestandteile** | **mg/100mL** |
|---|---|
| Tiotropiumbromid | 297,1 |
| Salmeterol x ½ H₂SO₄ | 276,7 |
| Benzalkoniumchlorid | 10 |
| Natriumedetat | 10 |
| Salzsäure (aq) | ad pH 2,9 |
| Wasser | ad 100mL |

Verwendung der Lösung im Respimat führt zu einer Dosierung von 20µg pro Dosierung **1** und 25µg/Dosierung **2.**
4) Lösung zur Anwendung im Respimat®:

| **Bestandteile** | **mg/100mL** |
|---|---|
| Tiotropiumbromid | 297,1 |
| Salmeterol x ½ H₂SO₄ | 276,7 |
| Benzalkoniumchlorid | 10 |
| Salzsäure (aq) | ad pH 2,9 |
| Wasser | ad 100mL |

Verwendung der Lösung im Respimat führt zu einer Dosierung von 20µg pro Dosierung **1** und 25µg/Dosierung **2.**
5) Lösung zur Anwendung im Respimat®:

| **Bestandteile** | **mg/100mL** |
|---|---|
| Tiotropiumbromid | 148,5 |
| Salmeterol x ½ H₂SO₄ | 1106,3 |
| Benzalkoniumchlorid | 8 |
| Natriumedetat | 50 |
| Salzsäure (aq) | ad pH 2,5 |
| Wasser | ad 100mL |

Verwendung der Lösung im Respimat führt zu einer Dosierung von 10µg pro Dosierung **1** und 100µg/Dosierung **2.**
6) Lösung zur Anwendung im Respimat®:

| **Bestandteile** | **mg/100mL** |
|---|---|
| Tiotropiumbromid | 1,5 |
| Salmeterol x ½ H₂SO₄ | 276,7 |
| Benzalkoniumchlorid | 8 |
| Natriumedetat | 10 |
| Salzsäure (aq) | ad pH 2,5 |
| Wasser | ad 100mL |

Verwendung der Lösung im Respimat führt zu einer Dosierung von 0,1µg pro Dosierung **1** und 25µg/Dosierung **2.**
7) Lösung zur Anwendung im Respimat®:

| **Bestandteile** | **mg/100mL** |
|---|---|
| Tiotropiumbromid | 14,9 |
| Salmeterol x ½ H₂SO₄ | 1106,32 |
| Benzalkoniumchlorid | 10 |
| Natriumedetat | 50 |
| Salzsäure (aq) | ad pH 3,5 |
| Wasser | ad 100mL |

Verwendung der Lösung im Respimat führt zu einer Dosierung von 1µg pro Dosierung **1** und 100µg/Dosierung **2**.
8) Lösung zur Anwendung im Respimat®:

| **Bestandteile** | **mg/100mL** |
|---|---|
| Tiotropiumbromid | 1486,1 |
| Salmeterol x ½ H₂SO₄ | 1106,32 |
| Benzalkoniumchlorid | 10 |
| Natriumedetat | 10 |
| Salzsäure (aq) | ad pH 3,5 |
| Wasser | ad 100mL |

Verwendung der Lösung im Respimat führt zu einer Dosierung von 100µg pro Dosierung **1** und 100µg/Dosierung **2.**
9) Konzentrierte Lösung:

| **Bestandteile** | **mg/100mL** |
|---|---|
| Tiotropiumbromid | 1486,1 |
| Salmeterol x ½ H₂SO₄ | 11063,2 |
| Benzalkoniumchlorid | 20 |
| Natriumedetat | 100 |
| Salzsäure (aq) | ad pH 3,5 |
| Wasser | ad 100mL |

## Patentansprüche

1. Treibgasfreie Inhalationslösung die als Lösemittel Wasser, Ethanol oder ein Gemisch aus Wasser und Ethanol enthält **gekennzeichnet durch** einen Gehalt an einem oder mehreren Tiotropiumsalzen (**1**) in Kombination mit einem oder mehreren Salmeterolsalzen (**2**), gegebenenfalls in Form ihrer Enantiomere, Gemische der Enantiomere oder in Form der Racemate, gegebenenfalls in Form der Solvate oder Hydrate sowie gegebenenfalls gemeinsam mit einem pharmazeutisch verträglichen Hilfsstoff, wobei das oder die Salze (**2**) des Salmeterols ausgewählt sind aus der Gruppe bestehend aus Hydrochlorid, Hydrobromid, Sulfat, Phosphat und Methansulfonat.

2. Inhalationslösung nach Anspruch 1, **dadurch gekennzeichnet, daß** die Wirkstoffe **1** und **2** entweder gemeinsam in einer einzigen Darreichungsform oder in zwei getrennten Darreichungsformen enthalten sind.

3. Inhalationslösung nach einem der Ansprüche 1 oder 2, **dadurch gekennzeichnet, daß 1** in Form des Chlorids, Bromids, lodids, Methansulfonats, para-Toluolsulfonats oder Methylsulfats, bevorzugt in Form des Bromids enthalten ist.

4. Inhalationslösung nach einem der Ansprüche 1 bis 3 , **dadurch gekennzeichnet, daß 2** ausgewählt ist aus den Salzen Hydrochlorid und Sulfat.

5. Inhalationslösung nach einem der Ansprüche 1 bis 4, **dadurch gekennzeichnet, daß 2** für Salmeterol x ½H₂SO₄ steht.

6. Inhalationslösung nach einem der Ansprüche 1 bis 5, **dadurch gekennzeichnet, daß** die Gewichtsverhältnisse von Tiotropium **1'** zu Salmeterol **2'** ,in einem Bereich von 1:300 bis 30:1, bevorzugt von 1:230 bis 20:1 liegen.

7. Inhalationslösung nach einem der Ansprüche 1 bis 6, **dadurch gekennzeichnet, daß** eine einmaliger Applikation einer Dosierung der Wirkstoffkombination **1'** und **2'** von 0,01 bis 1000µg, bevorzugt von 0,1 bis 200µg entspricht.

8. Inhaltionslösung nach einem der Ansprüche 1 bis 7, **dadurch gekennzeichnet, daß** der pH der Lösung 2 - 7, bevorzugt 2 - 5 beträgt.

9. Inhaltionslösung nach Anspruch 8, **dadurch gekennzeichnet, daß** der pH mittels einer Säure ausgewählt aus der Gruppe bestehend aus Salzsäure, Bromwasserstoffsäure, Salpetersäure, Schwefelsäure, Ascorbinsäure, Zitronensäure, Äpfelsäure, Weinsäure, Maleinsäure, Bernsteinsäure, Fumarsäure, Essigsäure, Ameisensäure und Propionsäure oder Gemischen davon, eingestellt wird.

10. Inhalationslösung nach einem der Ansprüche 1 bis 9, **dadurch gekennzeichnet, daß** sie gegebenenfalls weitere Co-Solventien und/oder Hilfsstoffe enthalten.

11. Inhalationslösung nach Anspruch 10, **dadurch gekennzeichnet, daß** sie als Co-Solventien Bestandteile enthalten, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester.

12. Inhalationslösung nach einem der Ansprüche 10 oder 11, **dadurch gekennzeichnet, daß** sie als Hilfsstoffe oberflächenaktive Stoffe Stabilisatoren, Komplexbildner, Antioxidantien und/oder Konservierungsstoffe, Geschmackstoffe, pharmakologisch unbedenkliche Salze und/oder Vitamine enthalten.

13. Inhalationslösungen nach Anspruch 12, **dadurch gekennzeichnet, daß** sie als Komplexbildner Editinsäure oder ein Salz der Editinsäure, bevorzugt Natriumedetat, enthalten.

14. Inhalationslösungen nach Anspruch 12 oder 13, dadurch gekennzeichent, daß sie als Antioxidantien,Verbindungen ausgewählt aus der Gruppe bestehend aus Ascorbinsäure, Vitamin A, Vitamin E und Tocopherole enthalten.

15. Inhalationslösungen nach Anspruch 12, 13 oder 14, **dadurch gekennzeichnet, daß** sie als Konservierungsmittel Verbindungen ausgewählt aus Cetylpyridiniumchlorid, Benzalkoniumchlorid, Benzoesäure und Benzoaten enthalten.

16. Inhalationslösungen nach einem der Ansprüche 10 bis 15, **dadurch gekennzeichnet, daß** sie neben den Wirkstoffen **1** und **2** und dem Lösemittel nur noch Bezalkoniumchlorid und Natriumedetat enthalten.

17. Inhalationslösungen nach einem der Ansprüche 10 bis 15, **dadurch gekennzeichnet, daß** sie neben den Wirkstoffen **1** und **2** und dem Lösemittel nur noch Benzalkoniumchlorid enthalten.

18. Inhalationslösungen nach einem der Ansprüche **1** bis **17, dadurch gekennzeichnet, daß** es sich um Konzentrate oder sterile gebrauchsfertige Inhalationslösungen handelt.

19. Verwendung einer Zusammensetzung nach einem der Ansprüche 1 bis 18 zur Herstellung eines Medikaments zur Behandlung von Atemwegserkrankungen.

20. Verwendung nach Anspruch 19 zur Herstellung eines Medikaments zur Behandlung von Asthma oder COPD.

## Claims

1. Propellant-free inhalable solution which contains as solvent water, ethanol or a mixture of water and ethanol, **characterised in that** it contains one or more tiotropium salts (**1**) combined with one or more salmeterol salts (**2**), optionally in the form of the enantiomers, mixtures of enantiomers or in the form of the racemates thereof, optionally in the form of the solvates or hydrates and optionally together with a pharmaceutically acceptable excipient, the salt or salts (**2**) of salmeterol being selected from the group consisting of hydrochloride, hydrobromide, sulphate, phosphate and methanesulphonate.

2. Inhalable solution according to claim 1, **characterised in that** the active substances **1** and **2** are contained either together in a single preparation or in two separate preparations.

3. Inhalable solution according to one of claims 1 or 2, **characterised in that 1** is contained in the form of the chloride, bromide, iodide, methanesulphonate, *para*-toluenesulphonate or methylsulphate, preferably in the form of the bromide.

4. Inhalable solution according to one of claims 1 to 3, **characterised in that 2** is selected from the hydrochloride and sulphate salts.

5. Inhalable solution according to one of claims 1 to 4, **characterised in that 2** denotes salmeterol x ½H₂SO₄.

6. Inhalable solution according to one of claims 1 to 5, **characterised in that** the ratios by weight of tiotropium **1'** to salmeterol **2'** are in a range from 1:300 to 30:1, preferably from 1:230 to 20:1.

7. Inhalable solution according to one of claims 1 to 6, **characterised in that** a single administration corresponds to a dosage of the active substance combination **1'** and **2'** of from 0.01 to 1000 µg, preferably from 0.1 to 200µg.

8. Inhalable solution according to one of claims 1 to 7, **characterised in that** the pH of the solution is 2 - 7, preferably 2 - 5.

9. Inhalable solution according to claim 8, **characterised in that** the pH is adjusted by means of an acid selected from among hydrochloric acid, hydrobromic acid, nitric acid, sulphuric acid, ascorbic acid, citric acid, malic acid, tartaric acid, maleic acid, succinic acid, fumaric acid, acetic acid, formic acid and propionic acid or mixtures thereof.

10. Inhalable solution according to one of claims 1 to 9, **characterised in that** it optionally contains other co-solvents and/or adjuvants.

11. Inhalable solution according to claim 10, **characterised in that** it contains, as co-solvents, ingredients which contain hydroxyl groups or other polar groups, e.g. alcohols - particularly isopropylalcohol, glycols - particularly propyleneglycol, polyethyleneglycol, polypropyleneglycol, glycolether, glycerol, polyoxyethylene alcohols and polyoxyethylene fatty acid esters.

12. Inhalable solution according to one of claims 10 or 11, **characterised in that** it contains as adjuvants surfactants, stabilisers, complexing agents, antioxidants and/or preservatives, flavourings, pharmacologically harmless salts and/or vitamins.

13. Inhalable solutions according to claim 12, **characterised in that** they contain editic acid or a salt of editic acid, preferably sodium edetate, as complexing agent.

14. Inhalable solutions according to claim 12 or 13, **characterised in that** they contain as antioxidants compounds selected from among ascorbic acid, vitamin A, vitamin E and tocopherols.

15. Inhalable solutions according to claim 12, 13 or 14, **characterised in that** they contain as preservatives compounds selected from among cetylpyridinium chloride, benzalkonium chloride, benzoic acid and benzoates.

16. Inhalable solutions according to one of claims 10 to 15, **characterised in that** they contain only benzalkonium chloride and sodium edetate in addition to the active substances **1** and **2** and the solvent.

17. Inhalable solutions according to one of claims 10 to 15, **characterised in that** they contain only benzalkonium chloride in addition to the active substances **1** and **2** and the solvent.

18. Inhalable solutions according to one of claims 1 to 17, **characterised in that** they are concentrates or sterile ready-to-use inhalable solutions.

19. Use of a composition according to one of claims 1 to 18 for preparing a medicament for the treatment of respiratory diseases.

20. Use according to claim 19 for preparing a medicament for the treatment of asthma or COPD.

## Revendications

1. Solution pour inhalation sans gaz propulseur qui contient comme solvant de l'eau, de l'éthanol ou un mélange d'eau et d'éthanol **caractérisée par** une teneur en un ou plusieurs sels de tiotropium (1) en combinaison avec un ou plusieurs sels de salmétérol (2), éventuellement sous forme de leurs énantiomères, de mélanges des énantiomères ou sous forme des racémates, éventuellement sous forme des solvates ou hydrates et éventuellement conjointement avec un adjuvant pharmaceutiquement acceptable, où le ou les sels (2) du salmétérol sont choisis dans le groupe consistant en le chlorhydrate, le bromhydrate, le sulfate, le phosphate et le méthanesulfonate.

2. Solution pour inhalation selon la revendication 1 **caractérisée en ce que** les principes actifs 1 et 2 sont contenus soit conjointement dans une forme d'administration unique soit dans deux formes d'administration séparées.

3. Solution pour inhalation selon l'une des revendications 1 ou 2 **caractérisée en ce que** 1 est contenu sous forme du chlorure, bromure, iodure, méthanesulfonate, para-toluènesulfonate ou méthylsulfate, de préférence sous forme du bromure.

4. Solution pour inhalation selon l'une des revendications 1 à 3 **caractérisée en ce que** 2 est choisi parmi les sels chlorhydrate et sulfate.

5. Solution pour inhalation selon l'une des revendications 1 à 4 **caractérisée en ce que** 2 est le salmétérol x ½ H₂SO₄.

6. Solution pour inhalation selon l'une des revendications 1 à 5 **caractérisée en ce que** les rapports massiques du tiotropium 1' au salmétérol 2' sont situés dans une plage de 1 : 300 à 30 : 1, de préférence de 1 : 230 à 20 : 1.

7. Solution pour inhalation selon l'une des revendications 1 à 6 **caractérisée en ce qu'**une application unique correspond à une posologie de la combinaison de principes actifs 1' et 2' de 0,01 à 1000 µg, de préférence de 0,1 à 200 µg.

8. Solution pour inhalation selon l'une des revendications 1 à 7 **caractérisée en ce que** le pH de la solution est 2-7, de préférence 2-5.

9. Solution pour inhalation selon la revendication 8 **caractérisée en ce que** le pH est ajusté au moyen d'un acide choisi dans le groupe consistant en l'acide chlorhydrique, l'acide bromhydrique, l'acide nitrique, l'acide sulfurique, l'acide ascorbique, l'acide citrique, l'acide malique, l'acide tartrique, l'acide maléique, l'acide succinique, l'acide fumarique, l'acide acétique, l'acide formique et l'acide propionique ou des mélanges de ceux-ci.

10. Solution pour inhalation selon l'une des revendications 1 à 9 **caractérisée en ce qu'**elle contient éventuellement d'autres co-solvants et/ou adjuvants.

11. Solution pour inhalation selon la revendication 10 **caractérisée en ce qu'**elle contient comme co-solvants des constituants qui contiennent des groupes hydroxyle ou d'autres groupes polaires, par exemple des alcools - en particulier l'alcool isopropylique, des glycols - en particulier le propylèneglycol, le polyéthylèneglycol, le polypropylèneglycol, les éthers de glycol, le glycérol, des polyoxyéthylènealcools et des esters de polyoxyéthylène-acides gras.

12. Solutions pour inhalation selon l'une des revendications 10 ou 11 **caractérisées en ce qu'**elles contiennent comme adjuvants des substances tensioactives, des stabilisants, des complexants, des antioxydants et/ou des conservateurs, des agents de sapidité, des sels pharmacologiquement acceptables et/ou des vitamines.

13. Solutions pour inhalation selon la revendication 12 **caractérisées en ce qu'**elles contiennent comme complexant l'acide édétique ou un sel de l'acide édétique, de préférence l'édétate de sodium.

14. Solutions pour inhalation selon la revendication 12 ou 13 **caractérisées en ce qu'**elles contiennent comme antioxydants des composés choisis dans le groupe consistant en l'acide ascorbique, la vitamine A, la vitamine E et les tocophérols.

15. Solutions pour inhalation selon la revendication 12, 13 ou 14 **caractérisées en ce qu'**elles contiennent comme conservateurs des composés choisis parmi le chlorure de cétylpyridinium, le chlorure de benzalkonium, l'acide benzoïque et les benzoates.

16. Solutions pour inhalation selon l'une des revendications 10 à 15 **caractérisées en ce qu'**elles ne contiennent, outre les principes actifs 1 et 2 et le solvant, que du chlorure de benzalkonium et de l'édétate de sodium.

17. Solutions pour inhalation selon l'une des revendications 10 à 15 **caractérisées en ce qu'**elles ne contiennent, outre les principes actifs 1 et 2 et le solvant, que du chlorure de benzalkonium.

18. Solutions pour inhalation selon l'une des revendications 1 à 17 **caractérisées en ce qu'**il s'agit de concentrés ou de solutions pour inhalation prêtes à l'emploi stériles.

19. Utilisation d'une composition selon l'une des revendications 1 à 18 pour la production d'un médicament pour le traitement des maladies des voies respiratoires.

20. Utilisation selon la revendication 19 pour la production d'un médicament pour le traitement de l'asthme ou de la COPD.
